# FASCICULE DE BREVET EUROPEEN

(11) **EP 0 858 319 B1**
(45) Date de publication et mention de la délivrance du brevet: **23.07.2003**
(21) Numéro de dépôt: 96934960.4
(22) Date de dépôt: 23.10.1996
(51) Int. Cl.: A61K 7/48

(54) **UTILISATION D'ACIDES CARBOXYLIQUES PORTEURS D'UNE FONCTION SOUFR E POUR FAVORISER LA DESQUAMATION DE LA PEAU OU STIMULER LE RENOUVELLEMENT EPIDERMIQUE**
VERWENDUNG VON CARBONSÄUREN MIT EINER SCHWEFELHALTIGEN FUNKTIONELLEN GRUPPE ZUR FÖRDERUNG DER HAUTABSCHÄLUNG BZW. STIMULIERUNG DER EPIDERMISREGENERATION
USE OF CARBOXYLIC ACIDS HAVING A SULPHUR FUNCTION FOR PROMOTING SKIN EXFOLIATION OR STIMULATING EPIDERMAL REGENERATION

(30) Priorité: 30.10.1995 FR 9512787
(43) Date de publication de la demande: 19.08.1998
(73) Titulaire: L'OREAL, 75008 Paris (FR)
(72) Inventeur: MAIGNAN, Jean, F-93290 Tremblay-en-France (FR)
(74) Mandataire: Corizzi, Valérie
(86) Numéro de dépôt international: FR9601658
(87) Numéro de publication internationale: WO97016165

(56) Documents cités:
- EP-A- 0 576 287
- CH-A- 497 175
- DE-A- 1 667 902
- FR-A- 1 605 354
- FR-A- 2 011 940
- FR-A- 2 201 087
- FR-A- 2 446 277
- FR-A- 2 476 484
- GB-A- 1 161 349
- GB-A- 1 163 870
- GB-A- 1 180 192
- GB-A- 2 023 003
- GB-A- 2 025 415
- GB-A- 2 040 683
- GB-A- 2 067 565
- GB-A- 2 077 620
- US-A- 3 629 452
- US-A- 3 671 643
- US-A- 4 035 492

## Description

L'invention se rapporte à l'utilisation d'acides carboxyliques porteurs d'une fonction thio-éther, d'une fonction sulfoxyde ou d'une fonction sulfone, dans ou pour la fabrication d'une composition cosmétique et/ou dermatologique pour favoriser la desquamation de la peau et/ou lutter contre le vieillissement intrinsèque et extrinsèque de la peau. Elle se rapporte aussi à un procédé de traitement non thérapeutique de la peau en vue de desquamer la peau ainsi qu'à un procédé de traitement du vieillissement cutané.

Le vieillissement cutané résultant d'effets sur la peau de facteurs intrinsèques ou extrinsèques, se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté et par l'apparition de télangiectasies.

Certains de ces signes du vieillissement sont plus particulièrement liés au vieillissement intrinsèque ou physiologique, c'est-à-dire au vieillissement « normal » lié à l'âge, alors que d'autres sont plus spécifiques du vieillissement extrinsèque, c'est-à-dire du vieillissement provoqué d'une manière générale par l'environnement ; il s'agit plus particulièrement du photo-vieillissement dû à l'exposition au soleil, à la lumière ou à tout autre rayonnement.

L'invention s'intéresse au vieillissement intrinsèque ou physiologique ainsi qu'au vieillissement extrinsèque.

Les changements de la peau dus au vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique.

Au contraire, le vieillissement extrinsèque entraîne des altérations cliniques telles que des rides épaisses et la formation d'une peau molle et tannée, et des changements histopathologiques tels qu'une excessive accumulation de matière élastique dans le derme supérieur et une dégénérescence des fibres de collagène.

On connaît dans l'art antérieur divers agents destinés à lutter contre le vieillissement cutané.

Ainsi, le brevet US-A-4 603 146 décrit l'emploi d'acide rétinoïque et de ses dérivés dans des compositions cosmétiques, en vue de lutter contre le vieillis-sement cutané. Par ailleurs, de nombreux brevets et publications (voir par exemple la demande EP-A-0413528) ainsi que de nombreuses compositions cosmétiques du commerce enseignent l'emploi des α-hydroxyacides comme l'acide lactique, l'acide glycolique ou encore l'acide citrique pour traiter le vieillissement cutané.

On connaît enfin les bêta-hydroxy-acides et plus spécialement l'acide salicylique ainsi que ses dérivés pour leur propriétés desquamantes (voir les documents WO-A-93/10756 et US-A-4 767 750).

Tous ces composés ont une action contre le vieillissement de la peau, consistant en une desquamation, c'est-à-dire l'élimination des cellules « mortes » situées à la surface du *stratum corneum.* Cette propriété desquamante est aussi appelée, souvent à tort, propriété kératolytique. Mais ces composés présentent également des effets secondaires, qui consistent en des picotements, des tiraillements, des échauffements et des rougeurs désagréables pour l'utilisateur.

On constate donc que subsiste le besoin d'agents antivieillissement ayant une action au moins aussi efficace que celle des composés de l'art antérieur, mais ne présentant pas leurs inconvénients.

La demanderesse a trouvé de manière inattendue que l'application topique de certains acides carboxyliques porteurs d'une fonction thio-éther, d'une fonction sulfoxyde ou d'une fonction sulfone, permettait de desquamer la peau ainsi que de stimuler le renouvellement cellulaire épidermique et la réparation épidermique.
Certes, les acides carboxyliques porteurs d'une fonction thio-éther, d'une fonction sulfoxyde ou d'une fonction sulfone sont déjà connus dans le domaine cosmétique dans des compositions à usage topique ou pour administration par voie orale, destinée à améliorer l'élasticité de la peau (EP-A-0576287), à traiter l'état gras des cheveux (GB-A-2025415, FR-A-2446277, GB-A-2067565, GB-A-2023003, GB-A-2040683, US-A-4,035,492), à lutter contre la séborrhée (GB-A-1161349, US-A-3,629,452, CH-A-497175, DE-A1667902, FR-A1605354, GB-A1,163870), à hydrater la peau (GB-A-2077620, FR-A-2476484), ou à traiter l'acné (FR-A2011940, US-A-3,671,643). Cependant personne jusqu'à ce jour n'a envisagé ni suggéré d'utiliser ces acides pour la desquamation de la peau, la stimulation du renouvellement épidermique ou le traitement du vieillissement cutané.
Aussi, la présente invention a pour objet l'utilisation d'acides carboxyliques porteurs d'une fonction thio-éther, d'une fonction sulfoxyde ou d'une fonction sulfone à l'état libre ou au moins partiellement neutralisé, dans ou pour la fabrication d'une composition cosmétique ou dermatologique pour favoriser la desquamation de la peau et/ou stimuler le renouvellement épidermique.
La présente invention a également pour objet l'utilisation d'acides carboxyliques porteurs d'une fonction thio-éther, d'une fonction sulfoxyde ou d'une fonction sulfone à l'état libre ou au moins partiellement neutralisé, comme défini dans la revendication 2, dans ou pour la fabrication d'une composition cosmétique ou dermatologique comme agent antivieillissement, notamment pour lutter contre les rides et/ou les ridules et/ou les taches actiniques et/ou les dyschromies cutanées et/ou les dermites et/ou les cicatrices.
L'invention a plus particulièrement pour objet l'utilisation d'au moins un acide carboxylique porteur d'une fonction thio-éther, d'une fonction sulfoxyde ou d'une fonction sulfone, à l'état libre ou au moins partiellement neutralisé, dans ou pour la fabrication d'une composition cosmétique ou dermatologique pour favoriser la desquamation de la peau et/ou stimuler le renouvellement épidermique, cet acide répondant à l'une des formules (I), (II) ou (III) suivantes : 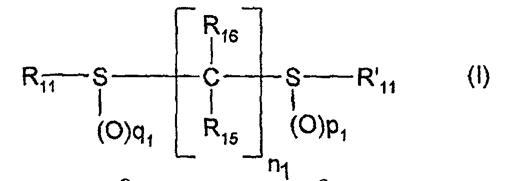 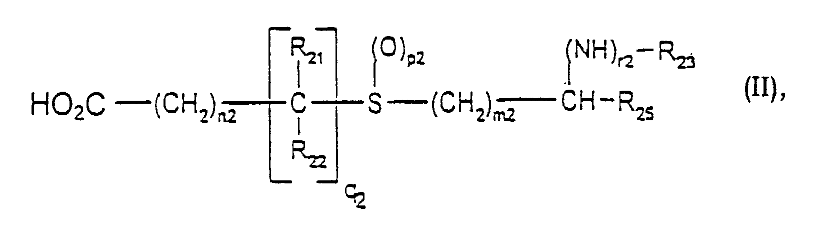 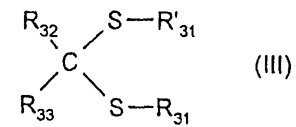 dans lesquelles n₁ représente un nombre entier d'une valeur allant de 1 à 10, p₁ et q₁, identiques ou différents, représentent 0, 1 ou 2, n₂ représente un nombre entier d'une valeur allant de 0 à 5, p₂ représente 0, 1 ou 2, q₂ représente 0, 1, 2 ou 3, m₂ représente un nombre entier d'une valeur allant de 0 à 10, r₂ représente 0 ou 1, q₂ et n₂ n'étant jamais tous deux nuls simultanément,
R₁₁ et R'₁₁, identiques ou différents, représentent un radical choisi dans le groupe constitué par :
a) 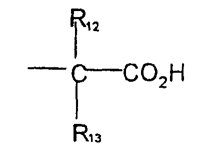
b) 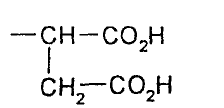
c) 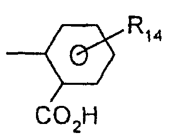
dans lesquels, R₁₂ et R₁₃ identiques ou différents sont choisis parmi le groupe constitué de l'atome d'hydrogène et des radicaux alcoyle ou alcényle linéaires ou ramifiés en C₁-C₈ ; R₁₄ est choisi parmi le groupe constitué de l'atome d'hydrogène, des radicaux alcoyles en C₁-C₈ linéaires ou ramifiés, des radicaux alcényles en C₁-C₈ linéaires ou ramifiés et des radicaux alcoxyles en C₁-C₈ linéaires ou ramifiés ; R₁₅ et R₁₆ sont choisis parmi le groupe constitué de l'atome d'hydrogène et des radicaux alcoxyles en C₁-C₈ linéaires ou ramifiés ; R₂₁ et R₂₂ sont choisis parmi le groupe constitué de l'atome d'hydrogène et des radicaux alcoxyles et alcényles en C₁-C₆ linéaires ou ramifiés, du phényle, des radicaux acide alcoylcarboxylique en C₁-C₆ et acide alcénylcarboxylique en C₁-C₆; R₂₃ représente un radical alcoyle ou alcényle linéaire ou ramifié en C₁-C₁₇ ou un radical acyle -CO-R₂₄, avec R₂₄ radical alcoyle ou alcényle linéaire ou ramifié en C₁-C₁₇; R₂₅ est choisi parmi l'atome d'hydrogène, les radicaux alcoyle et alcényle linéaires ou ramifiés en C₁-C₆ un radical acide alcoylcarboxylique en C₁-C₅, acide alcénylcarboxylique en C₁-C₅ et la fonction -COOH ; R₃₁ et R'₃₁ sont choisis parmi le groupe constitué des radicaux : 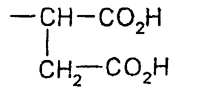 et

-CH₂-CO₂H

R₃₂ représente un radical choisi dans le groupe constitué de :

i) -CH₃

ii) -CO₂H

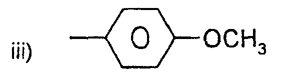

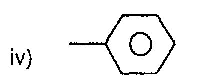

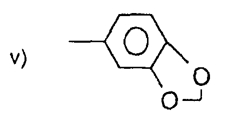

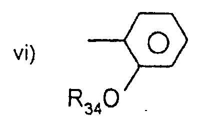

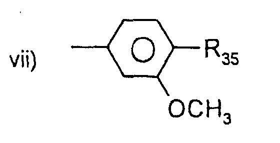

tel que R₃₄ représente un radical choisi parmi : -CH₂-CO₂H et -CO-CH₃
et R₃₅ représente un radical choisi parmi : OH, OCH₃, OC(CH₃)₃, OCH₂CO₂H
R₃₃ représente un radical choisi parmi le groupe constitué de :
l'atome d'hydrogène, un radical alcoyle ou alcényle linéaire ou ramifié en C₁-C₆, ou R₃₂ et R₃₃ forment ensemble un radical alcane di-yle -(CH₂)m₃- tel que m₃ représente un entier d'une valeur allant de 3 à 5, ou R₃₂ et R₃₃ forment ensemble un radical aralcane di-yle 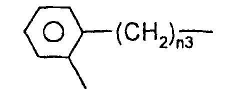 tel que n₃ représente 3 ou 4.

Les composés utilisables selon l'invention comprennent également les mono et les di-sels des produits décrits ci-dessus, ces sels pouvant être choisis parmi les sels alcalins, alcalino-terreux et les sels d'amines organiques. On citera à titre d'exemple les sels de sodium, les sels de potassium, de magnésium, de calcium, de strontium, les sels d'éthanolamine, de diéthanolamine, de triéthanolamine, les sels de lysine, d'arginine.

La desquamation de la peau est associée à une amélioration clinique de la qualité de la peau qui devient plus éclatante, moins ridée et d'une façon générale plus jeune. De plus, l'utilisation des acides décrits ci-dessus permet de traiter les imperfections de la peau telles que les taches, les dyschromies cutanées, les dermites, les lentigos actiniques, les cicatrices et les pigmentations cicatricielles.

Aussi, l'invention a encore pour objet l'utilisation d'au moins un acide carboxylique porteur d'une fonction thio-éther, d'une fonction sulfoxyde ou d'une fonction sulfone et notamment un acide répondant à l'une des formules (I), (II) ou (III) à l'état libre ou au moins partiellement neutralisé, dans ou pour la fabrication d'une composition cosmétique ou dermatologique comme agent antivieillissement, notamment pour lutter contre les rides et/ou les ridules et/ou les taches actiniques et/ou les dyschromies cutanées et/ou les dermites et/ou les cicatrices.

La demanderesse a en particulier constaté, sans que cela soit complètement expliqué, que ces composés ont une action antivieillissement au moins aussi efficace que les composés de l'art antérieur et que cette action est plus douce dans la mesure où aucune irritation n'est ressentie et aucune rougeur n'est observée lors de l'application sur la peau d'une composition cosmétique ou dermatologique les contenant.

De façon préférentielle, les acides de l'invention seront choisis parmi :
a) les composés répondant à la formule (I) dans laquelle
   R₁₅ = R₁₆ = H, p₁ = q₁ et R₁₁ = R'₁₁,
b) les composés répondant à la formule (II) dans laquelle :
   r₂ = 1, R₂₃ = H, R₂₅ = -COOH et m = 1 (dérivés de la cystéine) ou m = 2 (dérivés de l'homocystéine)
c) les composés répondant à la formule (III) dans laquelle :
   R₃₂ est un radical aromatique, R₃₃ = H et R₃₁ = R'₃₁.

En particulier, les acides de l'invention sont choisis parmi :
l'acide méthylène dithio-2,2' dibenzoïque,
l'acide éthylène dithio-2,2' dibenzoïque,
l'acide éthylène disulfinyl-2,2' dibenzoïque,
l'acide (propane di-yl-1,3 dithio)-2,2' dibenzoïque,
l'acide (butane di-yl-1,4 dithio)-2,2' dibenzoïque,
l'acide (pentane di-yl-1,5 dithio)-2,2' dibenzoïque,
l'acide (hexane di-yl-1,6 dithio)-2,2' dibenzoïque,
l'acide (hexane di-yl-1,6 disulfinyl)-2,2' dibenzoïque,
l'acide (hexane di-yl-1,6 dithio)-α,α' disuccinique,
l'acide (octane di-yl-1,8 dithio)-2,2' dibenzoïque,
l'acide (decane di-yl-1,10 dithio)-2,2' dibenzoïque,
l'acide (decane di-yl-1,10 dithio)-α,α' disuccinique,
l'acide méthylène dithio-α,α' diacétique,
l'acide éthylène dithio-α,α' diacétique,
l'acide éthylène disulfinyl-α,α' diacétique,
l'acide éthylène disulfonyl-α,α' diacétique,
l'acide (hexane di-yl-1,6 dithio)-α,α' diacétique,
l'acide (hexane di-yl-1,6 disulfinyl)-α,α' diacétique,
l'acide (hexane di-yl-1,6 disulfonyl)-α,α' diacétique,
l'acide (decane di-yl-1,10 dithio)-α,α' diacétique,
l'acide (decane di-yl-1,10 disulfinyl)-α,α' diacétique,
l'acide (decane di-yl-1,10 disulfonyl)-α,α' diacétique,
la S-(carboxyméthyl)-cystéine,
la S-(carboxyéthyl)-cystéine,
la S-(carboxy-4 butyl)-cystéine,
la S-(carboxyméthyl)-sulfinyl cystéine,
la S-(carboxyméthyl)-sulfonyl cystéine,
la S-(carboxy-1 éthyl-1)-cystéine,
la S-(carboxy-2 propyl-2)-cystéine,
l'acide (p-méthoxy benzylidène dithio)-α,α' disuccinique,
l'acide (benzylidène dithio)-α,α' disuccinique,
l'acide (pipéronylidène dithio)-α,α' disuccinique,
l'acide (vanillylidène dithio)-α,α' disuccinique,
l'acide (vératrylidène dithio)-α,α' disuccinique,
l'acide (butoxy-4 méthoxy-3 benzylidène dithio)-α,α' disuccinique,
l'acide (carboxyméthyloxy-4 méthoxy-3 benzylidène dithio)-α,α' disuccinique,
l'acide (p-méthoxy benzylidène dithio)-α,α' diacétique,
l'acide (benzylidène dithio)-α,α' diacétique,
l'acide (pipéronylidène dithio)-α,α' diacétique,
l'acide (vanillylidène dithio)-α,α' diacétique,
l'acide (vératrylidène dithio)-α,α' diacétique,
ainsi que leurs mono- et leurs di-sels de base minérale ou organique.

De préférence on utilisera selon l'invention la S-carboxyméthylcystéine ainsi que ses mono- et ses di-sels de base minérale ou organique.
La préparation des produits utilisés selon l'invention est bien connue de l'homme du métier :

Pour la synthèse des produits répondant à la formule (I), on pourra se référer au document FR-2447189 dans lequel cette synthèse est décrite avec R₁₅ = R₁₆ = H. On utilise les mêmes méthodes de synthèse dans les cas où R₁₅ et R₁₆ représentent d'autres radicaux.
La synthèse des produits répondant à la formule (II) avec p₂ = 0 est décrite dans les documents FR-1505874 et FR-1472021. Lorsque p₂ = 1, 2, les produits répondant à la formule (II) sont préparés à partir des thioéthers correspondants par un traitement oxydant selon des méthodes connues de l'homme du métier. On pourra, par exemple, se reporter aux méthodes d'oxydation des thioéthers en sulfoxydes et en sulfones décrites par Jerry March dans « Advanced Organic Chemistry », Wiley Interscience, 3è édition p1089.
La synthèse des produits répondant à la formule (III) a été décrite dans le document FR-2468362.

Dans les compositions selon l'invention, l'acide ou le mélange d'acides carboxyliques porteurs d'une fonction thio-éther, d'une fonction sulfoxyde ou d'une fonction sulfone et notamment les produits selon les formules (I) (II) et (III) peut être utilisé en une quantité allant de 0,2 à 20 % en poids par rapport au poids total de la composition et en particulier en une quantité allant de 0,5 à 10 % et mieux de 0,5 à 5 % en poids par rapport au poids total de la composition.

Les acides de l'invention peuvent être associés à d'autres actifs connus pour leurs propriétés desquamantes, comme les hydroxy-acides, les α- ou β-céto-acides, les rétinoïdes. Une telle association permet de diminuer la concentration active de ces demiers du fait des effets additifs. On peut ainsi obtenir une composition moins irritante et moins toxique ainsi qu'une composition plus efficace que celles de l'art antérieur n'utilisant que ces actifs.

Les hydroxyacides peuvent être par exemple des α-hydroxyacides ou des β-hydroxyacides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Ces hydroxyacides sont notamment les acides glycolique, lactique, malique, tartrique, citrique et de manière générale les acides de fruits, les acides hydroxy-2 alcanoïque, mandélique, salicylique, ainsi que leurs dérivés alkylés ou acylés comme l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthyl-benzoïque ou encore leurs dérivés alcoxylés comme l'acide 2-hydroxy-3-méthoxybenzoïque.

Les rétinoïdes peuvent être notamment l'acide rétinoïque (all-trans ou 13-cis) et ses dérivés, le rétinol (vitamine A) et ses esters tels que le palmitate de rétinol, l'acétate de rétinol et le propionate de rétinol ainsi que leurs sels, ou encore le rétinal.

A titre d'exemple, les hydroxyacides, les céto-acides et les rétinoïdes peuvent être utilisés dans les compositions selon l'invention en une quantité représentant de 0,1 à 5 % en poids du poids total de la composition et mieux de 0,5 à 3 %.

En vue de lutter efficacement contre le photovieillissement, il est en outre possible d'ajouter à la composition de l'invention un ou plusieurs filtres solaires complémentaires, actifs dans l'UVA et/ou l'UVB, hydrophiles ou lipophiles.

Un test *in vitro* d'efficacité de la desquamation a été réalisé sur kératinocytes en utilisant de l'acide n-octanoyl-5-salicylique (composé 1), de la S-carboxyméthylcystéine (composé 2), de l'ester bis-(1-éthoxycarbonyl-éthyl)- de l'acide nonane-dioique (composé 3), de l'acide 2-acétoxy-5-octanoyl-benzoique (composé 4) et de l'acide 5-oxothiomorpholine-3-carboxylique (composé 5).

Le principe du test repose sur le fait que la desquamation induit la libération de cornéocytes. Le pouvoir de desquamation du produit testé va être d'autant plus grand que le nombre de cornéocytes libérés sera important.

Le protocole du test a été le suivant : à partir de biopsies de peau, on a obtenu, par séparation de l'épiderme, des kératinocytes que l'on a dissociés par action enzymatique à la trypsine et mis en culture à la concentration de 2.10⁻⁵ cellules/ml. La croissance et la différenciation des kératinocytes ont été obtenues par culture durant 10 à 20 jours en milieu spécifique.

Puis, après élimination du milieu de culture, on a ajouté le produit à tester et évalué l'activité du produit. Pour ce faire, on a réalisé deux prélèvements à T₀ et T₆₀, c'est-à-dire avant l'ajout du produit et 60 minutes après cet ajout, et on a analysé les prélèvements ainsi effectués au cytomètre de flux pour dénombrer la population de cornéocytes. Au cytomètre de flux, les populations de cornéocytes et de kératinocytes sont différenciées par traitement à l'acridine orange spécifique de l'ADN des cellules, qui se lie au noyau des cellules et rèvèle donc exclusivement la présence des kératinocytes.

L'indice de détachement cellulaire est déterminé par la différence entre T₆₀ et T₀.

La même mesure a été réalisée pour un témoin ne contenant pas de produit à tester car l'expérience produit inévitablement la libération de cornéocytes, même en l'absence d'actif. La variation du témoin a fixé arbitrairement la norme de 100 %.

Les résultats sont rassemblés dans le tableau ci-dessous :

| Témoin | Composé 1 | Composé 2 | Composé 3 | Composé 4 | Composé 5 |
|---|---|---|---|---|---|
| 0 % | 106 % | 121 % | 59 % | inactif | inactif |

Ces résultats montrent clairement que la S-carboxyméthylcystéine, à concentration égale à celle de l'acide n-octanoyl-5-salicylique connu comme étant un actif desquamant puissant, est beaucoup plus actif que celui-ci, et que les autres composés ont des activités plus faibles que celle de S-carboxyméthylcystéine.

L'invention a encore pour objet un procédé de traitement non thérapeutique de la peau destiné à la desquamation de la peau consistant à appliquer sur la peau une composition contenant au moins un acide carboxylique porteur d'une fonction thio-éther, d'une fonction sulfoxyde ou d'une fonction sulfone et notamment un acide répondant à l'une des formules (I), (II) ou (III), à l'état libre ou partiellement neutralisé, dans un milieu cosmétiquement et/ou dermatologiquement acceptable.

L'invention a aussi pour objet un procédé de traitement cosmétique ou dermatologique du vieillissement de la peau, consistant à appliquer sur la peau une composition contenant au moins un acide tel que défini ci-dessus, dans un milieu cosmétiquement et/ou dermatologiquement acceptable.

La composition de l'invention contient un milieu cosmétiquement ou dermatologiquement acceptable, c'est-à-dire un milieu compatible avec la peau, les ongles, les muqueuses, les tissus et les cheveux. La composition contenant l'acide porteur d'une fonction thio-éther, d'une fonction sulfoxyde ou d'une fonction sulfone peut être appliquée par voie topique sur le visage, le cou, les cheveux, les muqueuses et les ongles ou toute autre zone cutanée du corps.

Les compositions selon l'invention peuvent se présenter sous toutes les formes appropriées pour une application topique, notamment sous forme de solutions aqueuses, hydroalcooliques ou huileuses, de dispersions du type lotion ou sérum, de gels aqueux, anhydres ou huileux, d'émulsions de consistance liquide ou semi-liquide du type lait, obtenues par dispersion d'une phase grasse dans une phase aqueuse (H/E) ou inversement (E/H), de suspensions ou d'émulsions de consistance molle, semi-solide ou solide du type crème, gel, de microémulsions, ou encore de microcapsules, de microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage de la peau.

Les compositions peuvent également consister en des préparations solides constituant des savons ou des pains de nettoyage.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la quantité d'huile peut aller jusqu'à plus de 90 % en poids du poids total de la composition.

De façon connue, la composition de l'invention peut contenir également des adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les agents séquestrants, les charges et les matières colorantes. Les quantités de ces différents adjuvants sont celles classiquement utilisées dans les domaines considérés, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (huile de karité, huile d'amande douce), les huiles animales, les huiles de synthèse, les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer le Polysorbate 60 et le stéarate de sorbitane vendus respectivement sous les dénominations commerciales Tween 60 et Span 60 par la Société ICI. On peut aussi utiliser le stéarate de PEG 20, commercialisé par la société ICI sous la marque MYRJ.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropyl-cellulose, les gommes naturelles (xanthane) et les argiles, et, comme gélifiants lipophiles, on peut citer les argiles modifiées comme les bentones, les sels métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon, les extraits bactériens ou végétaux, notamment d'Aloe Vera, des antiseptiques.

Comme actifs lipophiles, on peut utiliser le tocophérol (vitamine E) et ses dérivés, les acides gras essentiels, les céramides, les huiles essentielles.

On peut, entre autre, associer les acides à des agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que la vitamine D et ses dérivés, les oestrogènes tels que l'oestradiol, l'acide kojique ou l'hydroquinone;
- les agents anti-radicaux libres, tels que l'alpha-tocophérol ou ses esters, les superoxyde dismutases, certains chélatants de métaux ou l'acide ascorbique et ses esters.

On peut, par ailleurs, associer aux acides de l'invention des antagonistes de substance P et/ou de CGRP (Calcitonin Gene Related Peptide ou peptide lié au gène de la calcitonine) tels que l'Iris Pallida et les sels de strontium, notamment les chlorures et les nitrates de strontium, ou des antagonistes de substance P et/ou de CGRP tels que ceux décrits dans les demandes de brevet français déposées au nom de la demanderesse sous les numéros 9405537 et 9500900. Une telle association permet de garantir une tolérance parfaite de ces compositions, même par des peaux très sensibles.

Le procédé de traitement cosmétique ou dermatologique de l'invention peuvent être mis en oeuvre notamment en appliquant les compositions hygiéniques, cosmétiques ou dermatologiques telles que définies ci-dessus, selon la technique d'utilisation habituelle de ces compositions. Par exemple : application de crèmes, de gels, de sérums, de pommades, de lotions, de laits sur la peau, le cuir chevelu, les ongles et/ou les muqueuses.

Les exemples suivants illustrent l'invention. Dans ces exemples, les proportions indiquées sont des pourcentages en poids.

### Exemple 1 : Lotion stabilisante

| | |
|---|---|
| S-carboxyméthylcystéine | 2 % |
| Bactéricide | 0,1 % |
| Tri-éthanolamine | 1,7 % |
| Agent séquestrant | 0,1 % |
| Transcutol (Gattefosse) | 5 % |
| Ethanol | 10,6 % |
| eau | 80,5 % |

On obtient une lotion, qui, appliquée quotidiennement sur la peau du visage, permet d'observer une amélioration de la souplesse et de l'élasticité de la peau traitée.

### Exemple 2 : Crème purifiante non teintée

| | |
|---|---|
| Allantoine | 0,1 % |
| S-carboxyméthylcystéine | 1 % |
| Stéarate de glycérol | 1,2 % |
| Huiles | 12 % |
| Cire | 2 % |
| Conservateur | 0,3 % |
| Antiseptique | 0,2 % |
| Parfum | 0,4 % |
| Tri-éthanolamine | 0,8 % |
| Eau | qsp 100 |
| PEG 20 stéarate | 6,6 % |
| Ethanol | 4,2 % |

On obtient une crème qui, en application régulière, permet d'estomper les taches de la peau par desquamation.

### Exemple 3 : Lotion astringente

| | |
|---|---|
| S-carboxyméthylcystéine | 2 % |
| Colorant | 0,00008% |
| Parfum | 0,08 % |
| Alcool éthylique | 16,60 % |
| Conservateur | 0,20 % |
| Tri-éthanolamine | 1,5 % |
| Agent séquestrant | 0,1 % |
| Carbonate de magnésie précipité extra léger | 0,08 % |
| Eau | qsp 100 |
| Ethanol | 16,6 % |

L'application sous contrôle dermatologique de cette solution permet d'obtenir une desquamation profonde de la couche cornée et, ainsi, la mise en jeu d'un processus de réparation épidermique, ayant comme effet thérapeutique final un effacement des taches et dyschromies, un estompement des rides et ridules et une amélioration de l'état clinique de la peau, dont l'aspect devient celui d'une peau plus jeune.

Cette application se fait à raison d'une à trois séances hebdomadaires pendant 4 à 6 semaines.

### Exemple 4 : Lotion astringente

Cet exemple se différentie de l'exemple 3 par l'addition de 0,25% de nitrate de strontium. Cette formule convient particulièrement au traitement des peaux sensibles.

## Revendications

1. Utilisation cosmétique d'au moins un acide carboxylique porteur d'une fonction thio-éther, d'une fonction sulfoxyde ou d'une fonction sulfone, à l'état libre ou au moins partiellement neutralisé, dans une composition cosmétique topique, comme agent favorisant la desquamation de la peau et/ou stimulant le renouvellement épidermique.

2. Utilisation cosmétique d'au moins un acide carboxylique porteur d'une fonction thio-éther, d'une fonction sulfoxyde ou d'une fonction sulfone, à l'état libre ou au moins partiellement neutralisé, dans une composition cosmétique topique, comme agent destiné à lutter contre le vieillissement cutané, **caractérisée en ce que** l'acide carboxylique porteur d'une fonction thio-éther, d'une fonction sulfoxyde ou d'une fonction sulfone répond à l'une des formules (I), (II) ou (III) suivantes : 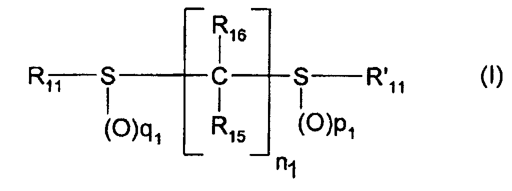 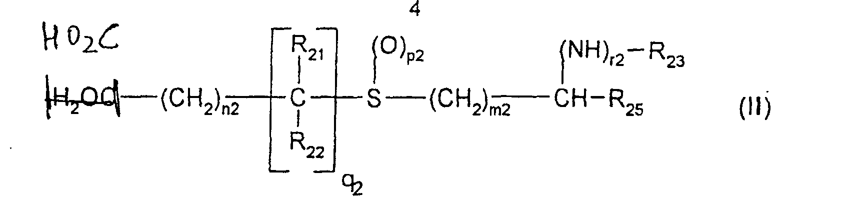 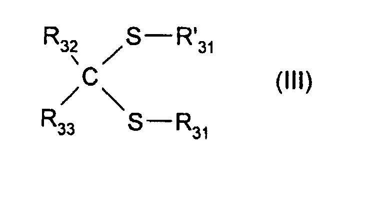 dans lesquelles n₁ représente un nombre entier d'une valeur allant de 1 à 10, p₁ et q₁, identiques ou différents, représentent 0, 1 ou 2, n₂ représente un nombre entier d'une valeur allant de 0 à 5, p₂ représente 0, 1 ou 2, q₂ représente 0, 1, 2 ou 3, m₂ représente un nombre entier d'une valeur allant de 0 à 10, r₂ représente 0 ou 1, q₂ et n₂ n'étant jamais tous deux nuls simultanément,
R₁₁ et R'₁₁, identiques ou différents, représentent un radical choisi dans le groupe constitué par :
a) 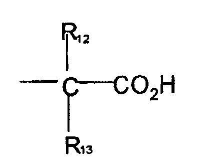
b) 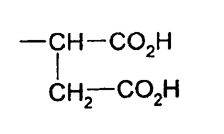
c) 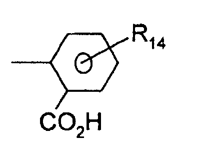
dans lesquels, R₁₂ et R₁₃ identiques ou différents sont choisis parmi le groupe constitué de l'atome d'hydrogène et des radicaux alcoyle ou alcényle linéaires ou ramifiés en C₁-C₈ ; R₁₄ est choisi parmi le groupe constitué de l'atome d'hydrogène, des radicaux alcoyles en C₁-C₈ linéaires ou ramifiés, des radicaux alcényles en C₁-C₈ linéaires ou ramifiés et des radicaux alcoxyles en C₁-C₈ linéaires ou ramifiés ; R₁₅ et R₁₆ sont choisis parmi le groupe constitué de l'atome d'hydrogène et des radicaux alcoxyles en C₁-C₈ linéaires ou ramifiés ; R₂₁ et R₂₂ sont choisis parmi le groupe constitué de l'atome d'hydrogène et des radicaux alcoxyles et alcényles en C₁-C₆ linéaires ou ramifiés, du phényle, des radicaux acide alcoylcarboxylique en C₁-C₆ et acide alcénylcarboxylique en C₁-C₆; R₂₃ représente un radical alcoyle ou alcényle linéaire ou ramifié en C₁-C₁₇ ou un radical acyle -CO-R₂₄, avec R₂₄ radical alcoyle ou alcényle linéaire ou ramifié en C₁-C₁₇; R₂₅ est choisi parmi l'atome d'hydrogène, les radicaux alcoyle et alcényle linéaires ou ramifiés en C₁-C₆ un radical acide alcoylcarboxylique en C₁-C₅, acide alcénylcarboxylique en C₁-C₅ et la fonction -COOH ; R₃₁ et R'₃₁ sont choisis parmi le groupe constitué des radicaux : 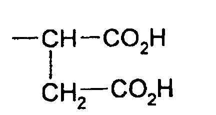 et
-CH₂-CO₂H
R₃₂ représente un radical choisi dans le groupe constitué de :
i) -CH₃
ii) -CO₂H 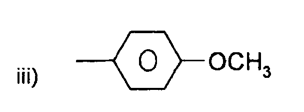 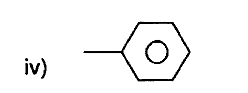 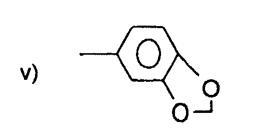 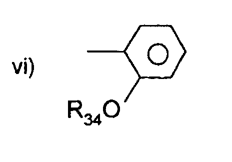 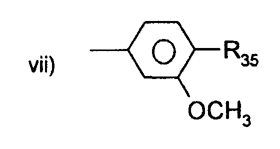
tel que R₃₄ représente un radical choisi parmi : -CH₂-CO₂H et -CO-CH₃
et R₃₅ représente un radical choisi parmi : OH, OCH₃, OC(CH₃)₃, OCH₂CO₂H
R₃₃ représente un radical choisi parmi le groupe constitué de :
l'atome d'hydrogène, un radical alcoyle ou alcényle linéaire ou ramifié en C₁-C₆, ou R₃₂ et R₃₃ forment ensemble un radical alcane di-yle -(CH₂)m₃- tel que m₃ représente un entier d'une valeur allant de 3 à 5, ou R₃₂ et R₃₃ forment ensemble un radical aralcane di-yle 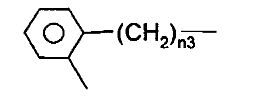 tel que n₃ représente 3 ou 4.

3. Utilisation d'au moins un acide carboxylique porteur d'une fonction thio-éther, d'une fonction sulfoxyde ou d'une fonction sulfone, à l'état libre ou au moins partiellement neutralisé, pour la fabrication d'une composition dermatologique destinée à favoriser la desquamation de la peau et/ou à stimuler le renouvellement épidermique.

4. Utilisation selon l'une des revendications 1 ou 3, **caractérisée en ce que** l'acide carboxylique porteur d'une fonction thio-éther, d'une fonction sulfoxyde ou d'une fonction sulfone répond à l'une des formules (I), (II) ou (III) suivantes : 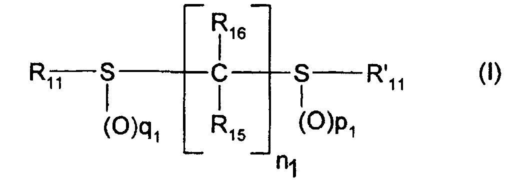 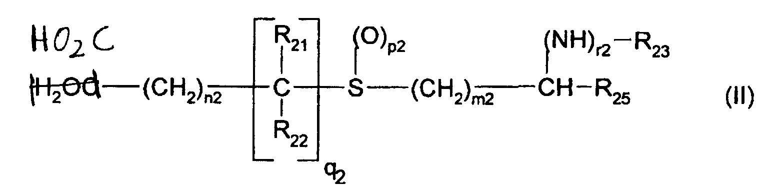 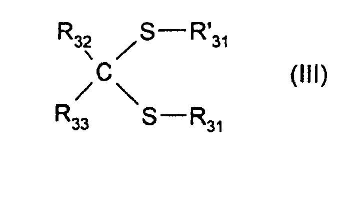 dans lesquelles n₁ représente un nombre entier d'une valeur allant de 1 à 10, p₁ et q₁, identiques ou différents, représentent 0, 1 ou 2, n₂ représente un nombre entier d'une valeur allant de 0 à 5, p₂ représente 0, 1 ou 2, q₂ représente 0, 1, 2 ou 3, m₂ représente un nombre entier d'une valeur allant de 0 à 10, r₂ représente 0 ou 1, q₂ et n₂ n'étant jamais tous deux nuls simultanément,
R₁₁ et R'₁₁, identiques ou différents, représentent un radical choisi dans le groupe constitué par :
a) 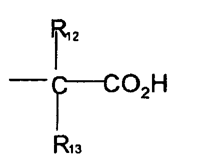
b) 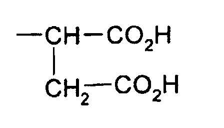
c) 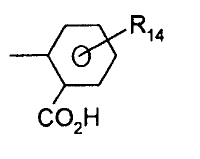
dans lesquels, R₁₂ et R₁₃ identiques ou différents sont choisis parmi le groupe constitué de l'atome d'hydrogène et des radicaux alcoyle ou alcényle linéaires ou ramifiés en C₁-C₈ ; R₁₄ est choisi parmi le groupe constitué de l'atome d'hydrogène, des radicaux alcoyles en C₁-C₈ linéaires ou ramifiés, des radicaux alcényles en C₁-C₈ linéaires ou ramifiés et des radicaux alcoxyles en C₁-C₈ linéaires ou ramifiés ; R₁₅ et R₁₆ sont choisis parmi le groupe constitué de l'atome d'hydrogène et des radicaux alcoxyles en C₁-C₈ linéaires ou ramifiés ; R₂₁ et R₂₂ sont choisis parmi le groupe constitué de l'atome d'hydrogène et des radicaux alcoxyles et alcényles en C₁-C₆ linéaires ou ramifiés, du phényle, des radicaux acide alcoylcarboxylique en C₁-C₈ et acide alcénylcarboxylique en C₁-C₆; R₂₃ représente un radical alcoyle ou alcényle linéaire ou ramifié en C₁-C₁₇ ou un radical acyle -CO-R₂₄, avec R₂₄ radical alcoyle ou alcényle linéaire ou ramifié en C₁-C₁₇; R₂₅ est choisi parmi l'atome d'hydrogène, les radicaux alcoyle et alcényle linéaires ou ramifiés en C₁-C₆ un radical acide alcoylcarboxylique en C₁-C₅, acide alcénylcarboxylique en C₁-C₅ et la fonction -COOH ; R₃₁ et R'₃₁ sont choisis parmi le groupe constitué des radicaux : 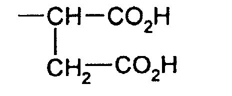 et
-CH₂-CO₂H
R₃₂ représente un radical choisi dans le groupe constitué de :
i) -CH₃
ii) -CO₂H 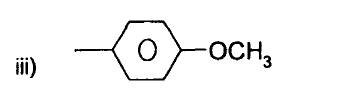 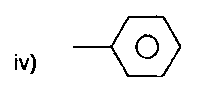 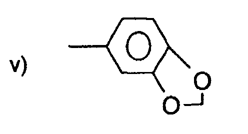 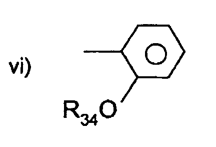 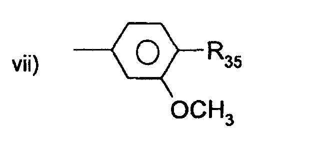
tel que R₃₄ représente un radical choisi parmi : -CH₂-CO₂H et -CO-CH₃
et R₃₅ représente un radical choisi parmi : OH, OCH₃, OC(CH₃)₃, OCH₂CO₂H
R₃₃ représente un radical choisi parmi le groupe constitué de :
l'atome d'hydrogène, un radical alcoyle ou alcényle linéaire ou ramifié en C₁-C₆, ou R₃₂ et R₃₃ forment ensemble un radical alcane di-yle -(CH₂)m₃- tel que m₃ représente un entier d'une valeur allant de 3 à 5, ou R₃₂ et R₃₃ forment ensemble un radical aralcane di-yle 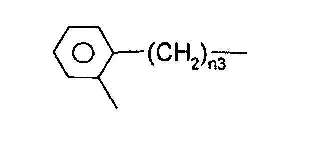 tel que n₃ représente 3 ou 4.

5. Utilisation selon l'une des revendications 1 à 4, **caractérisée en ce que** les acides sont choisis parmi :
a) les composés répondant à la formule (I) dans laquelle
R₁₅ = R₁₆ = H, p₁ = q₁ et R₁₁ = R'₁₁,
b) les composés répondant à la formule (II) dans laquelle :
r₂ = 1, R₂₃ = H, R₂₅ = -COOH et m = 1 (dérivés de la cystéine) ou m = 2 (dérivés de l'homocystéine)
c) les composés répondant à la formule (III) dans laquelle :
R₃₂ est un radical aromatique, R₃₃ = H et R₃₁ = R'₃₁.

6. Utilisation selon la revendication 5, **caractérisée en ce que** l'acide est choisi parmi :
l'acide méthylène dithio-2,2' dibenzoïque,
l'acide éthylène dithio-2,2' dibenzoïque,
l'acide éthylène disulfinyl-2,2' dibenzoïque,
l'acide (propane di-yl-1,3 dithio)-2,2' dibenzoïque,
l'acide (butane di-yl-1,4 dithio)-2,2' dibenzoïque,
l'acide (pentane di-yl-1,5 dithio)-2,2' dibenzoïque,
l'acide (hexane di-yl-1,6 dithio)-2,2' dibenzoïque,
l'acide (hexane di-yl-1,6 disulfinyl)-2,2' dibenzoïque,
l'acide (hexane di-yl-1,6 dithio)-α,α' disuccinique,
l'acide (octane di-yl-1,8 dithio)-2,2' dibenzoïque,
l'acide (decane di-yl-1,10 dithio)-2,2' dibenzoïque,
l'acide (decane di-yl-1,10 dithio)-α,α' disuccinique,
l'acide méthylène dithio-α,α' diacétique,
l'acide éthylène dithio-α,α' diacétique,
l'acide éthylène disulfinyl-α,α' diacétique,
l'acide éthylène disulfonyl-α,α' diacétique,
l'acide (hexane di-yl-1,6 dithio)-α,α' diacétique,
l'acide (hexane di-yl-1,6 disulfinyl)-α,α' diacétique,
l'acide (hexane di-yl-1,6 disulfonyl)-α,α' diacétique,
l'acide (decane di-yl-1,10 dithio)-α,α' diacétique,
l'acide (decane di-yl-1,10 disulfinyl)-α,α' diacétique,
l'acide (decane di-yl-1,10 disulfonyl)-α,α' diacétique,
la S-(carboxyméthyl)-cystéine,
la S-(carboxyéthyl)-cystéine,
la S-(carboxy-4 butyl)-cystéine,
la S-(carboxyméthyl)-sulfinyl cystéine,
la S-(carboxyméthyl)-sulfonyl cystéine,
la S-(carboxy-1 éthyl-1)-cystéine,
la S-(carboxy-2 propyl-2)-cystéine,
l'acide (p-méthoxy benzylidène dithio)-α,α' disuccinique,
l'acide (benzylidène dithio)-α,α' disuccinique,
l'acide (pipéronylidène dithio)-α,α' disuccinique,
l'acide (vanillylidène dithio)-α,α' disuccinique,
l'acide (vératrylidène dithio)-α,α' disuccinique,
l'acide (butoxy-4 méthoxy-3 benzylidène dithio)-α,α' disuccinique,
l'acide (carboxyméthyloxy-4 méthoxy-3 benzylidène dithio)-α,α' disuccinique,
l'acide (p-méthoxy benzylidène dithio)-α,α' diacétique,
l'acide (benzylidène dithio)-α,α' diacétique,
l'acide (pipéronylidène dithio)-α,α' diacétique,
l'acide (vanillylidène dithio)-α,α' diacétique,
l'acide (vératrylidène dithio)-α,α' diacétique,
ainsi que leurs mono- et leurs di-sels de base minérale ou organique.

7. Utilisation selon l'une des revendications 1 à 6, **caractérisée en ce que** l'acide est la S-carboxyméthyicystéine ou l'un de ses mono- et di-sels de base minérale ou organique.

8. Utilisation selon l'une des revendications 1 à 7, **caractérisée en ce que** l'acide est utilisé en une quantité allant de 0,2 à 20 % et de préférence de 0,5 à 5 % en poids par rapport au poids total de la composition.

9. Utilisation selon l'une des revendications 1 à 8, **caractérisée en ce que** la composition comprend, en outre, au moins un actif choisi parmi les α- ou β-hydroxy-acides, les α- ou β-céto-acides et les rétinoïdes.

10. Utilisation selon l'une des revendications 1 à 9, **caractérisée en ce que** que la composition comprend, en outre, au moins un actif choisi parmi les acides glycolique, lactique, malique, tartrique, citrique, hydroxy-2 alcanoïque, mandélique, salicylique, l'acide n-octanoyl-5-salicylique.

11. Utilisation selon la revendication 8 ou 9, **caractérisée en ce que** l'actif est présent en une quantité allant de 0,1 à 5 % en poids par rapport au poids total de la composition.

12. Utilisation selon l'une des revendications 1 à 11, **caractérisée en ce que** la composition contient, en outre, au moins un adjuvant choisi parmi les protéines ou les hydrolysats de protéine, les acides aminés, les polyols, l'urée, les sucres et les dérivés de sucre, les vitamines, l'amidon, les extraits végétaux, les acides gras essentiels, les céramides, les huiles essentielles.

13. Utilisation selon l'une des revendications 1 à 12, **caractérisée en ce que** la composition est une solution aqueuse, huileuse ou hydroalcoolique, une émulsion eau-dans-huile ou huile-dans-eau, une microémulsion, un gel aqueux ou anhydre, un sérum, une dispersion de vésicules, de microcapsules ou de microparticules.

14. Utilisation selon l'une des revendications 1 à 13, **caractérisée en ce que** la composition contient, en outre, au moins un antagoniste de substance P et/ou de CGRP.

15. Utilisation selon la revendication 14, **caractérisée en ce que** l'antagoniste est un sel de strontium.

## Patentansprüche

1. Kosmetische Verwendung mindestens einer Carbonsäure mit Thioethergruppe, Sulfoxidgruppe oder Sulfongruppe, die im freien Zustand vorliegt oder zumindest teilweise neutralisiert ist, in einer kosmetischen Zusammensetzung zur topischen Anwendung als Wirkstoff zur Förderung der Abschuppung der Haut und/oder Stimulierung der Epidermiserneurerung.

2. Kosmetische Verwendung mindestens einer Carbonsäure mit Thioethergruppe, Sulfoxidgruppe oder Sulfongruppe, die im freien Zustand vorliegt oder zumindest teilweise neutralisiert ist, in einer kosmetischen Zusammensetzung zur topischen Anwendung als Wirkstoff zur Bekämpfung der Hautalterung, **dadurch gekennzeichnet, dass** die Carbonsäure mit Thioethergruppe, Sulfoxidgruppe oder Sulfongruppe einer der folgenden Formeln (I), (II) oder (III) entspricht: 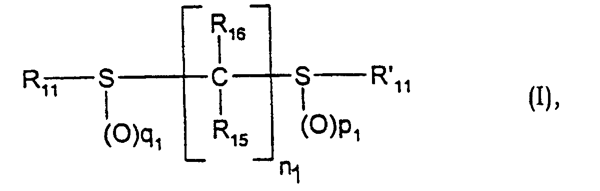 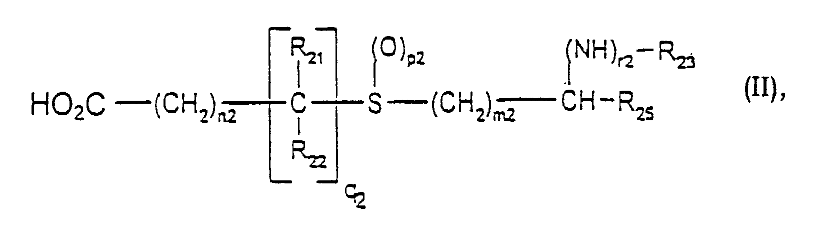 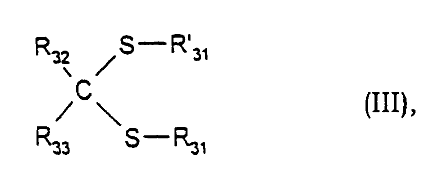 wobei in den Formeln n₁ eine ganze Zahl im Bereich von 1 bis 10 bedeutet, p₁ und q₁, die gleich oder verschieden sind, 0, 1 oder 2 bedeuten, n₂ 0 oder eine ganze Zahl im Bereich von 1 bis 5 bedeutet, p₂ 0, 1 oder 2 ist, q₂ 0, 1, 2 oder 3 bedeutet, m₂ 0 oder eine ganze Zahl im Bereich von 1 bis 10 ist und r₂ 0 oder 1 bedeutet, wobei q₂ und n₂ nicht beide gleichzeitig 0 sein können,
die Gruppen R₁₁ und R'₁₁, die gleich oder voneinander verschieden sind, bedeuten eine Gruppe, die ausgewählt ist unter:
a) 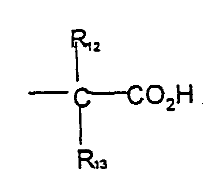
b) 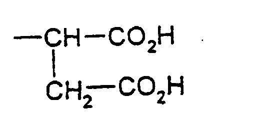
c) 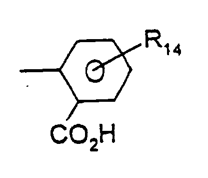
worin R₁₂ und R₁₃, die gleich oder verschieden sind, unter Wasserstoff und den geradkettigen oder verzweigten Alkyl- oder Alkenylgruppen mit 1 bis 8 Kohlenstoffatomen ausgewählt sind; und R₁₄ unter Wasserstoff, den geradkettigen oder verzweigten C₁₋₈-Alkylgruppen, den geradkettigen oder verzweigten C₁₋₈-Alkenylgruppen und den geradkettigen oder verzweigten C₁₋₈-Alkoxygruppen ausgewählt ist;
R₁₅ und R₁₆ sind unter Wasserstoff und geradkettigen oder verzweigten C₁₋₈-Alkoxygruppen ausgewählt;
R₂₁ und R₂₂ sind unter Wasserstoff und geradkettigen oder verzweigten Alkoxy- und Alkenylgruppen mit 1 bis 6 Kohlenstoffatomen, Phenyl, C₁₋₆-Alkylcarbonsäuregruppen und C₁₋₆-Alkenylcarbonsäuregruppen ausgewählt;
R₂₃ bedeutet eine geradkettige oder verzweigte Alkyl- oder Alkenylgruppe mit 1 bis 17 Kohlenstoffatomen oder eine Acylgruppe -CO-R₂₄, wobei R₂₄ eine geradkettige oder verzweigte Alkyl- oder Alkenylgruppe mit 1 bis 17 Kohlenstoffatomen ist;
R₂₅ ist unter Wasserstoff, geradkettigen oder verzweigten Alkylund Alkenylgruppen mit 1 bis 6 Kohlenstoffatomen, C₁₋₅-Alkylcarbonsäuregruppen, C₁₋₅-Alkenylcarbonsäuregruppen und der Gruppe -COOH ausgewählt;
R₃₁ und R'₃₁ sind unter den folgenden Gruppen ausgewählt: 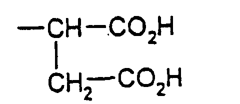 und
-CH₂-CO₂H;
R₃₂ bedeutet eine Gruppe, die unter den folgenden Verbindungen ausgewählt ist:
i) -CH₃
ii) -CO₂H 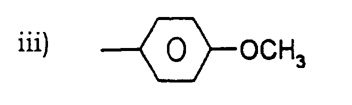 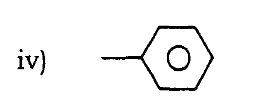 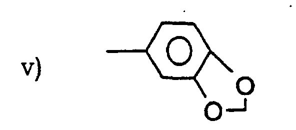 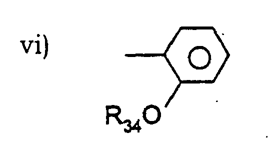 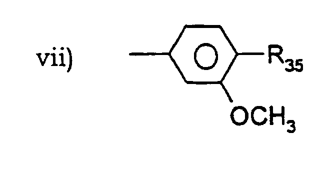
wobei R₃₄ eine Gruppe bedeutet, die unter -CH₂-CO₂H und -CO-CH₃ ausgewählt ist, und R₃₅ eine Gruppe bedeutet, die unter OH, OCH₃, OC(CH₃)₃ und OCH₂CO₂H ausgewählt ist;
R₃₃ bedeutet eine Gruppe, die unter den folgenden Verbindungen ausgewählt ist:
Wasserstoff, geradkettigen oder verzweigten Alkyl- oder Alkenylgruppen mit 1 bis 6 Kohlenstoffatomen, oder
R₃₂ und R₃₃ bilden gemeinsam eine Alkandiylgruppe -(CH₂)m₃-, wobei m₃ eine ganze Zahl im Bereich von 3 bis 5 bedeutet, oder R₃₂ und R₃₃ bilden gemeinsam eine Aralkandiylgruppe 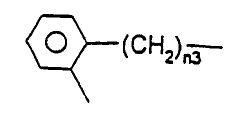 wobei n₃ 3 oder 4 bedeutet.

3. Verwendung mindestens einer Carbonsäure mit Thioethergruppe, Sulfoxidgruppe oder Sulfongruppe, die im freien Zustand vorliegt oder zumindest teilweise neutralisiert ist, zur Herstellung einer dermatologischen Zusammensetzung, die zur Förderung der Abschuppung der Haut und/oder zur Stimulierung der Epidermiserneuerung vorgesehen ist.

4. Verwendung nach einem der Ansprüche 1 oder 3, **dadurch gekennzeichnet, dass** die Carbonsäure mit Thioethergruppe, Sulfoxidgruppe oder Sulfongruppe einer der folgenden Formeln (I), (II) oder (III) entspricht: 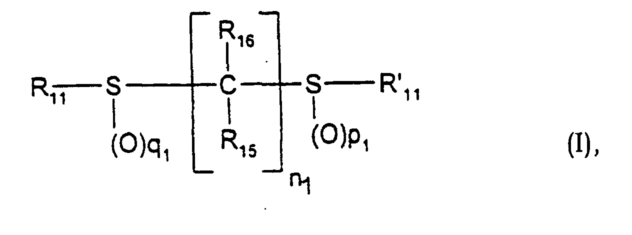 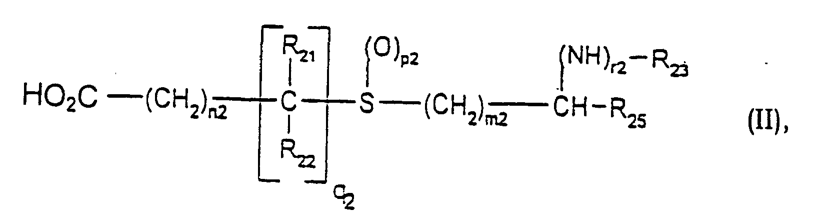 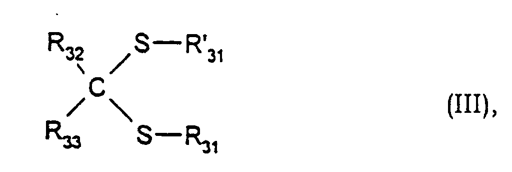 wobei in den Formeln n₁ eine ganze Zahl im Bereich von 1 bis 10 bedeutet, p₁ und q₁, die gleich oder verschieden sind, 0, 1 oder 2 bedeuten, n₂ 0 oder eine ganze Zahl im Bereich von 1 bis 5 bedeutet, p₂ 0, 1 oder 2 ist, q₂ 0, 1, 2 oder 3 bedeutet, m₂ 0 oder eine ganze Zahl im Bereich von 1 bis 10 ist und r₂ 0 oder 1 bedeutet, wobei q₂ und n₂ nicht beide gleichzeitig 0 sein können,
die Gruppen R₁₁ und R'₁₁, die gleich oder voneinander verschieden sind, bedeuten eine Gruppe, die ausgewählt ist unter:
a) 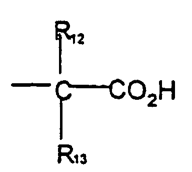
b) 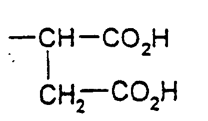
c) 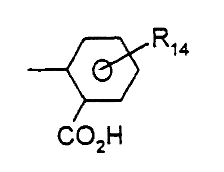
worin R₁₂ und R₁₃, die gleich oder verschieden sind, unter Wasserstoff und den geradkettigen oder verzweigten Alkyl- oder Alkenylgruppen mit 1 bis 8 Kohlenstoffatomen ausgewählt sind; und R₁₄ unter Wasserstoff, den geradkettigen oder verzweigten C₁₋₈-Alkylgruppen, den geradkettigen oder verzweigten C₁₋₈-Alkenylgruppen und den geradkettigen oder verzweigten C₁₋₈-Alkoxygruppen ausgewählt ist;
R₁₅ und R₁₆ sind unter Wasserstoff und geradkettigen oder verzweigten C₁₋₈-Alkoxygruppen ausgewählt;
R₂₁ und R₂₂ sind unter Wasserstoff und geradkettigen oder verzweigten Alkoxy- und Alkenylgruppen mit 1 bis 6 Kohlenstoffatomen, Phenyl, C₁₋₆-Alkylcarbonsäuregruppen und C₁₋₆-Alkenylcarbonsäuregruppen ausgewählt;
R₂₃ bedeutet eine geradkettige oder verzweigte Alkyl- oder Alkenylgruppe mit 1 bis 17 Kohlenstoffatomen oder eine Acylgruppe -CO-R₂₄, wobei R₂₄ eine geradkettige oder verzweigte Alkyl- oder Alkenylgruppe mit 1 bis 17 Kohlenstoffatomen ist;
R₂₅ ist unter Wasserstoff, geradkettigen oder verzweigten Alkylund Alkenylgruppen mit 1 bis 6 Kohlenstoffatomen, C₁₋₅-Alkylcarbonsäuregruppen, C₁₋₅-Alkenylcarbonsäuregruppen und der Gruppe -COOH ausgewählt;
R₃₁ und R'₃₁ sind unter den folgenden Gruppen ausgewählt: 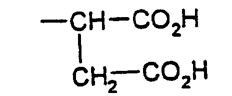 und
-CH₂-CO₂H;
R₃₂ bedeutet eine Gruppe, die unter den folgenden Verbindungen ausgewählt ist:
i) -CH₃
ii) -CO₂H 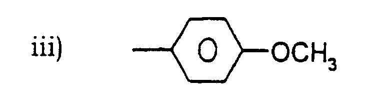 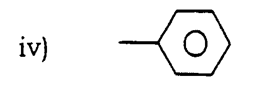 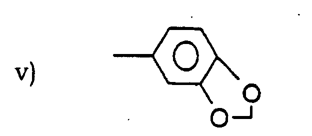 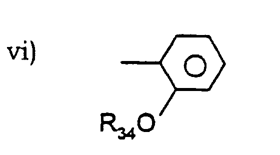 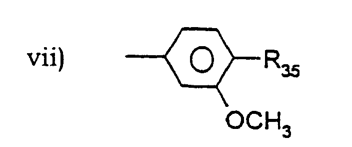
wobei R₃₄ eine Gruppe bedeutet, die unter -CH₂-COH und -CO-CH₃ ausgewählt ist, und R₃₅ eine Gruppe bedeutet, die unter OH, OCH₃, OC(CH₃)₃ und OCH₂CO₂H ausgewählt ist;
R₃₃ bedeutet eine Gruppe, die unter den folgenden Verbindungen ausgewählt ist:
Wasserstoff, geradkettigen oder verzweigten Alkyl- oder Alkenylgruppen mit 1 bis 6 Kohlenstoffatomen, oder
R₃₂ und R₃₃ bilden gemeinsam eine Alkandiylgruppe -(CH₂)m₃-, wobei m₃ eine ganze Zahl im Bereich von 3 bis 5 bedeutet, oder R₃₂ und R₃₃ bilden gemeinsam eine Aralkandiylgruppe 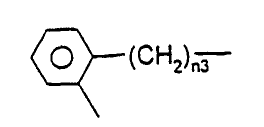 wobei n₃ 3 oder 4 bedeutet.

5. Verwendung nach einem der Ansprüche 1 bis 4, **dadurch gekennzeichnet, dass** die Säuren ausgewählt sind unter:
a) Verbindungen der Formel (I), worin bedeuten:
R₁₅ = R₁₆ = H, p₁ = q₁ und R₁₁ = R'₁₁,
b) Verbindungen der Formel (II), worin bedeuten:
r₂ = 1, R₂₃ = H, R₂₅ = -COOH und m = 1 (Cysteinderivate) oder m = 2 (Homocysteinderivate), und
c) Verbindungen der Formel (III), worin bedeuten:
R₃₂ eine aromatische Gruppe, R₃₃ = H und R₃₁ = R'₃₁.

6. Verwendung nach Anspruch 5, **dadurch gekennzeichnet, dass** die Säure unter den folgenden Verbindungen ausgewählt ist:
2,2'-Methylen-dithio-dibenzoesäure,
2,2'-Ethylen-dithio-dibenzoesäure,
2,2'-Ethylen-disulfinyl-dibenzoesäure,
2,2'-(1,3-Propandiyl-dithio)-dibenzoesäure,
2,2'-(1,4-Butandiyl-dithio)-dibenzoesäure,
2,2'-(1,5-Pentandiyl-dithio)-dibenzoesäure,
2,2'-(1,6-Hexandiyl-dithio)-dibenzoesäure,
2,2'-(1,6-Hexandiyl-disulfinyl)-dibenzoesäure,
α,α'-(1,6-Hexandiyl-dithio)-dibernsteinsäure,
2,2'-(1,8-Octandiyl-dithio)-dibenzoesäure,
2,2'-(1,10-Decandiyl-dithio)-dibenzoesäure,
α,α'-(1,10-Decandiyl-dithio) -dibernsteinsäure,
a,a'-Methylen-dithio-diessigsäure,
α,α'-Ethylen-dithio-diessigsäure,
α,α'-Ethylen-disulfinyl-diessigsäure,
α,α'-Ethylen-disulfonyl-diessigsäure,
α,α'-(1,6-Hexandiyl-dithio)-diessigsäure,
α,α'-(1,6-Hexandiyl-disulfinyl)-diessigsäure,
α,α'-(1,6-Hexandiyl-disulfonyl)-diessigsäure,
α,α'-(1,10-Decandiyl-dithio)-diessigsäure,
α,α'-(1,10-Decandiyl-disulfinyl)-diessigsäure,
α,α'-(1,10-Decandiyl-disulfonyl)-diessigsäure,
S-(Carboxymethyl)-cystein,
S-(Carboxyethyl)-cystein,
S-(4-Carboxybutyl)-cystein,
S-(Carboxymethyl)-sulfinyl-cystein,
S-(Carboxymethyl)-sulfonyl-cycstein,
S-(1-Carboxy-1-ethyl)-cystein,
S-(2-Carboxy-2-propyl)-cystein,
α,α'-(p-Methoxybenzyliden-dithio)-dibernsteinsäure,
α,α'-(Benzyliden-dithio)-dibernsteinsäure,
α,α'-(Piperonyliden-dithio)-dibernsteinsäure,
α,α'-(Vanillyliden-dithio)-dibernsteinsäure,
α,α'-(Veratryliden-dithio) -dibernsteinsäure,
α,α'-(4-Butoxy-3-methoxy-benzyliden-dithio)-dibernsteinsäure,
α,α'-(4-Carboxymethyloxy-3-methoxybenzyliden-dithio)-dibernsteinsäure,
α,α'-(p-Methoxybenzyliden-dithio)-diessigsäure,
α,α'-(Benzyliden-dithio)-diessigsäure,
α,α'-(Piperonyliden-dithio)-diessigsäure,
α,α'-(Vanillyliden-dithio)-diessigsäure und
α,α'-(Veratryliden-dithio)-diessigsäure,
sowie deren Mono- und Disalzen mit anorganischen oder organischen Basen.

7. Verwendung nach einem der Ansprüche 1 bis 6, **dadurch gekennzeichnet, dass** es sich bei der Säure um das S-Carboxymethylcystein oder seine Mono- und Disalze mit einer organischen oder anorganischen Base handelt.

8. Verwendung nach einem der Ansprüche 1 bis 7, **dadurch gekennzeichnet, dass** die Säure in einer Menge von 0,2 bis 20% und vorzugsweise 0,5 bis 5 Gew.-%, bezogen auf das Gesamtgewicht der Zusammensetzung, verwendet wird.

9. Verwendung nach einem der Ansprüche 1 bis 8, **dadurch gekennzeichnet, dass** die Zusammensetzung ferner mindestens einen Wirkstoff enthält, der unter den α- oder β-Hydroxysäuren, den α- oder β-Ketosäuren und den Retinoiden ausgewählt ist.

10. Verwendung nach einem der Ansprüche 1 bis 9, **dadurch gekennzeichnet, dass** die Zusammensetzung ferner mindestens einen Wirkstoff enthält, der unter Glykolsäure, Milchsäure, Äpfelsäure, Weinsäure, Citronensäure, 2-Hydroxyalkansäuren, Mandelsäure, Salicylsäure und 5-n-Octanoylsalicylsäure ausgewählt ist.

11. Verwendung nach Anspruch 8 oder 9, **dadurch gekennzeichnet, dass** der Wirkstoff in einer Menge von 0,1 bis 5 Gew.-%, bezogen auf das Gesamtgewicht der Zusammensetzung, vorliegt.

12. Verwendung nach einem der Ansprüche 1 bis 11, **dadurch gekennzeichnet, dass** die Zusammensetzung ferner mindestens einen Zusatzstoff enthält, der unter den Proteinen oder Proteinhydrolysaten, Aminosäuren, Polyolen, Harnstoff, Zuckern und Zuckerderivaten, Vitaminen, Stärke, Pflanzenextrakten, essentiellen Fettsäuren, Ceramiden oder etherischen Ölen ausgewählt ist.

13. Verwendung nach einem der Ansprüche 1 bis 12, **dadurch gekennzeichnet, dass** die Zusammensetzung eine wässrige, ölige oder wässrig-alkoholische Lösung, eine Wasser-in-Öl-Emulsion oder Öl-in-Wasser-Emulsion, eine Mikroemulsion, ein wässriges oder wasserfreies Gel, ein Serum, eine Vesikeldispersion oder eine Dispersion von Mikrokapseln oder Mikropartikeln ist.

14. Verwendung nach einem der Ansprüche 1 bis 13, **dadurch gekennzeichnet, dass** die Zusammensetzung ferner mindestens einen Antagonisten der Substanz P und/oder CGRP-Antagonisten enthält.

15. Verwendung nach Anspruch 14, **dadurch gekennzeichnet, dass** der Antagonist ein Strontiumsalz ist.

## Claims

1. Cosmetic use of at least one carboxylic acid carrying a thioether function, a sulphoxide function or a sulphone function, in the free state or at least partially neutralized, in a topical cosmetic composition, as an agent which promotes desquamation of the skin and/or promotes epidermal renewal.

2. Cosmetic use of at least one carboxylic acid carrying a thioether function, a sulphoxide function or a sulphone function, in the free state or at least partially neutralized, in a topical cosmetic composition, as an agent for combating cutaneous ageing, **characterized in that** the carboxylic acid carrying a thioether function, a sulphoxide function or a sulphone function corresponds to one of the following formulae (I), (II) or (III): 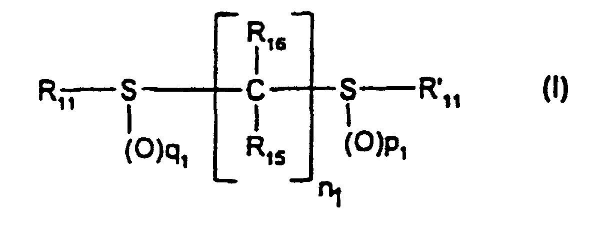 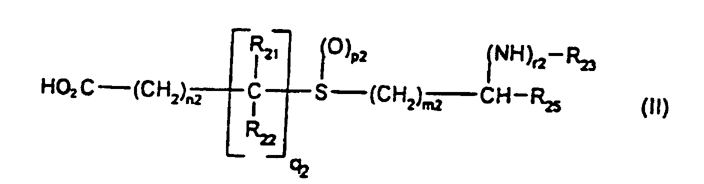 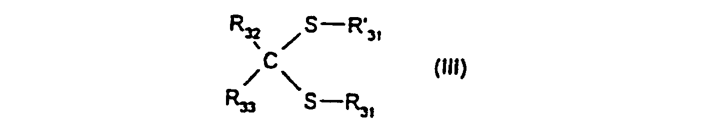 in which n₁ is an integer of a value ranging from 1 to 10, p₁ and q₁, which are identical or different, are 0, 1 or 2, n₂ is an integer of a value ranging from 0 to 5, p₂ is 0, 1 or 2, q₂ is 0, 1, 2 or 3, m₂ is an integer of a value ranging from 0 to 10, r₂ is 0 or 1, q₂ and n₂ never both being zero simultaneously,
R₁₁ and R'₁₁, which are identical or different, are a radical chosen from the group formed by:
**a)** 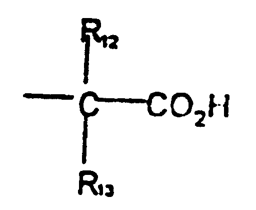
**b)** 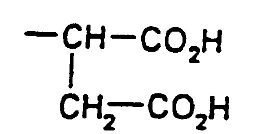
**c)** 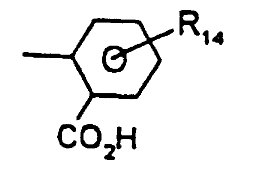
in which R₁₂ and R₁₃, which are identical or different, are chosen from the group formed by the hydrogen atom and C₁-C₈ linear or branched alkyl or alkenyl radicals; R₁₄ is chosen from the group formed by the hydrogen atom, linear or branched C₁-C₈ alkyl radicals, linear or branched C₁-C₈ alkenyl radicals and linear or branched C₁-C₈ alkoxy radicals; R₁₅ and R₁₆ are chosen from the group formed by the hydrogen atom and linear or branched C₁-C₈ alkoxy radicals; R₂₁ and R₂₂ are chosen from the group formed by the hydrogen atom and linear or branched C₁-C₆ alkoxy and alkenyl radicals, phenyl, C₁-C₆ alkylcarboxylic and C₁-C₆ alkenylcarboxylic acid radicals; R₂₃ is a C₁-C₁₇ linear or branched alkyl or alkenyl radical or an acyl radical -CO-R₂₄, where R₂₄ is a C₁-C₁₇ linear or branched alkyl or alkenyl radical; R₂₅ is chosen from the hydrogen atom, the C₁-C₆ linear or branched alkyl and alkenyl radicals, a C₁-C₅ alkylcarboxylic acid radical, C₁-C₅ alkenylcarboxylic acid radical and the function -COON; R₃₁ and R'₃₁ are chosen from the group formed by the radicals: 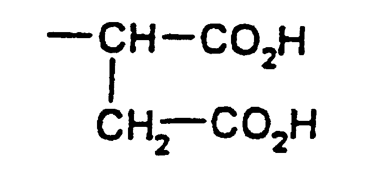 and
-CH₂-CO₂H
R₃₂ is a radical chosen from the group formed by:
i) -CH₃
ii) -CO₂H 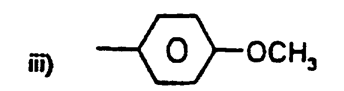 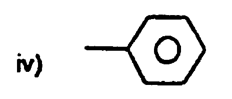 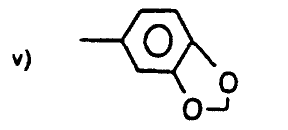 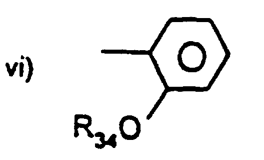 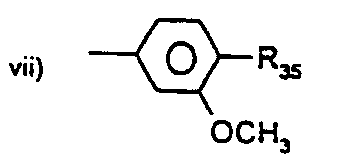
such that R₃₄ is a radical chosen from: -CH₂-CO₂H and -CO-CH₃
and R₃₅ is a radical chosen from: OH, OCH₃, OC(CH₃)₃, OCH₂CO₂H
R₃₃ is a radical chosen from among the group formed by:
the hydrogen atom, a C₁-C₆ linear or branched alkyl or alkenyl radical, or R₃₂ and R₃₃ together form an alkanediyl radical -(CH₂)m₃- such that m₃ is an integer of a value ranging from 3 to 5, or R₃₂ and R₃₃ together form an aralkanediyl radical 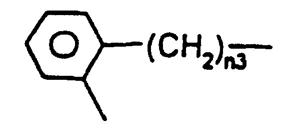 such that n₃ is 3 or 4.

3. Use of at least one carboxylic acid carrying a thioether function, a sulphoxide function or a sulphone function, in the free state or at least partially neutralized, for the production of a dermatological composition for promoting desquamation of the skin and/or promoting epidermal renewal.

4. Use according to either of Claims 1 and 3, **characterized in that** the carboxylic acid carrying a thioether function, a sulphoxide function or a sulphone function corresponds to one of the following formulae (I), (II) or (III): 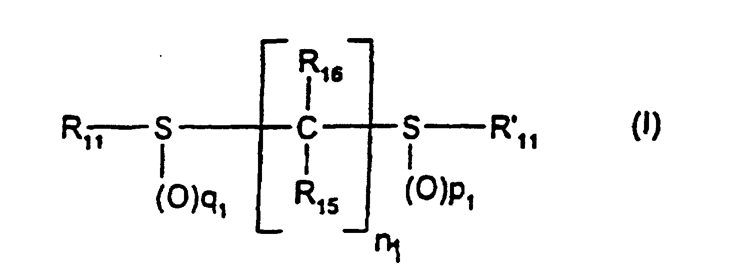 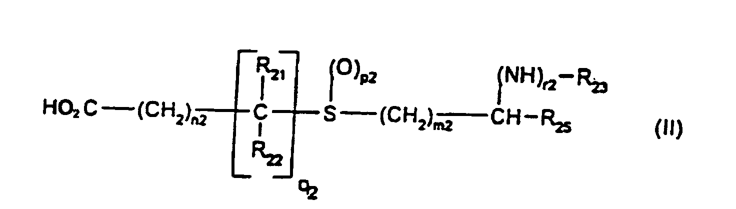 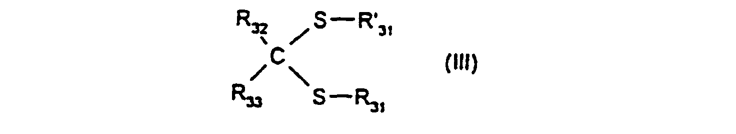 in which n₁ is an integer of a value ranging from 1 to 10, p₁ and q₁, which are identical or different, are 0, 1 or 2, n₂ is an integer of a value ranging from 0 to 5, p₂ is 0, 1 or 2, q₂ is 0, 1, 2 or 3, m₂ is an integer of a value ranging from 0 to 10, r₂ is 0 or 1, q₂ and n₂ never both being zero simultaneously,
R₁₁ and R'₁₁, which are identical or different, are a radical chosen from the group formed by:
**a)** 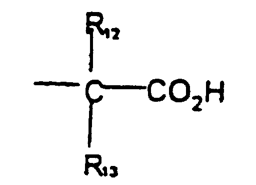
**b)** 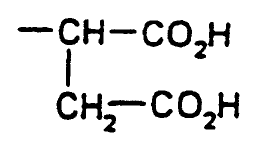
**c)** 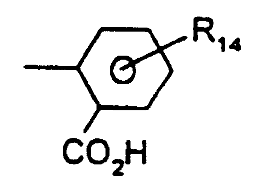
in which R₁₂ and R₁₃, which are identical or different, are chosen from the group formed by the hydrogen atom and C₁-C₈ linear or branched alkyl or alkenyl radicals; R₁₄ is chosen from the group formed by the hydrogen atom, linear or branched C₁-C₈ alkyl radicals, linear or branched C₁-C₈ alkenyl radicals and linear or branched C₁-C₈ alkoxy radicals; R₁₅ and R₁₆ are chosen from the group formed by the hydrogen atom and linear or branched C₁-C₈ alkoxy radicals; R₂₁ and R₂₂ are chosen from the group formed by the hydrogen atom and linear or branched C₁-C₆ alkoxy and alkenyl radicals, phenyl, C₁-C₆ alkylcarboxylic and C₁-C₆ alkenylcarboxylic acid radicals; R₂₃ is a C₁-C₁₇ linear or branched alkyl or alkenyl radical or an acyl radical -CO-R₂₄, where R₂₄ is a C₁-C₁₇ linear or branched alkyl or alkenyl radical; R₂₅ is chosen from the hydrogen atom, C₁-C₆ linear or branched alkyl and alkenyl radicals, a C₁-C₅ alkylcarboxylic acid radical, C₁-C₅ alkenylcarboxylic acid radical and the function -COOH; R₃₁ and R'₃₁ are chosen from the group formed by the radicals: 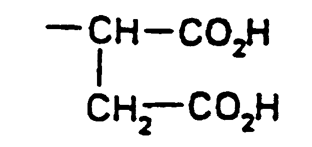 and
-CH₂-CO₂H
R₃₂ is a radical chosen from the group formed by:
i) -CH₃
ii) -CO₂H 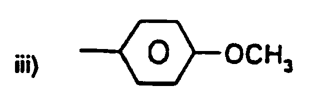 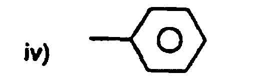 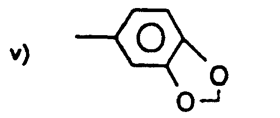 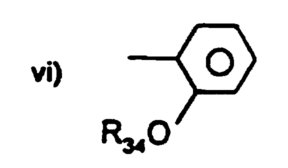 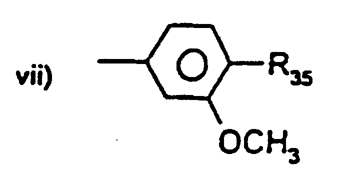
such that R₃₄ is a radical chosen from: -CH₂-CO₂H and -CO-CH₃
and R₃₅ is a radical chosen from: OH, OCH₃, OC(CH₃)₃, OCH₂CO₂H
R₃₃ is a radical chosen from among the group formed by:
the hydrogen atom, a C₁-C₆ linear or branched alkyl or alkenyl radical, or R₃₂ and R₃₃ together form an alkanediyl radical -(CH₂)m₃- such that m₃ is an integer of a value ranging from 3 to 5, or R₃₂ and R₃₃ together form an aralkanediyl radical 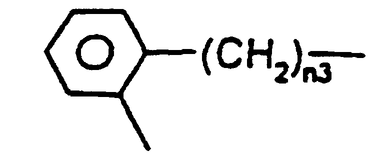 such that n₃ is 3 or 4.

5. Use according to one of Claims 1 to 4, **characterized in that** the acids are chosen from:
a) the compounds corresponding to the formula (I) in which
R₁₅ = R₁₆ = H, p₁ = q₁ and R₁₁ = R'₁₁,
b) the compounds corresponding to the formula (II) in which:
r₂ = 1, R₂₃ = H, R₂₅ = -COOH and m = 1 (cysteine derivatives) or m = 2 (homocysteine derivatives)
c) the compounds corresponding to the formula (III) in which:
R₃₂ is an aromatic radical, R₃₃ = H and R₃₁ = R'₃₁.

6. Use according to Claim 5, **characterized in that** the acid is chosen from:
2,2'-dithiomethylenedibenzoic acid,
2,2'-dithioethylenedibenzoic acid,
2,2'-disulphinylethylenedibenzoic acid,
2,2'-(dithiopropane-1,3-diyl)dibenzoic acid,
2,2'-(dithiobutane-1,4-diyl)dibenzoic acid,
2,2'-(dithiopentane-1,5-diyl)dibenzoic acid,
2,2'-(dithiohexane-1,6-diyl)dibenzoic acid,
2,2'-(disulphinylhexane-1,6-diyl)dibenzoic acid,
α,α'-(dithiohexane-1,6-diyl)disuccinic acid,
2,2'-(dithiooctane-1,8-diyl)dibenzoic acid,
2,2'-(dithiodecane-1,10-diyl)dibenzoic acid,
α,α'-(dithiodecane-1,10-diyl)disuccinic acid,
α,α'-dithiomethylenediacetic acid,
α,α'-dithioethylenediacetic acid,
α,α'-disulphinylethylenediacetic acid,
α,α'-disulphonylethylenediacetic acid,
α,α'-(dithiohexane-1,6-diyl)diacetic acid,
α,α'-(disulphinylhexane-1,6-diyl)diacetic acid,
α,α'-(disulphonylhexane-1,6-diyl)diacetic acid,
α,α'-(dithiodecane-1,10-diyl)diacetic acid,
α,α'-(disulphinyldecane-1,10-diyl)diacetic acid,
α,α'-(disulphonyldecane-1,10-diyl)diacetic acid,
S-(carboxymethyl)cysteine,
S-(carboxyethyl)cysteine,
S-(4-carboxybutyl)cysteine,
S-(carboxymethyl)sulphinylcysteine,
S-(carboxymethyl)sulphonylcysteine,
S-(1-carboxy-1-ethyl)cysteine,
S-(2-carboxy-2-propyl)cysteine,
α,α'-(p-methoxybenzylidenedithio)disuccinic acid,
α,α'-(benzylidenedithio)disuccinic acid,
α,α'-(piperonylidenedithio)disuccinic acid,
α,α'-(vanillylidenedithio)disuccinic acid,
α,α'-(veratrylidenedithio)disuccinic acid,
α,α'-(4-butoxy-3-methoxybenzylidenedithio)disuccinic acid,
α,α'-(4-carboxymethyloxy-3-methoxybenzylidenedithio)disuccinic acid,
α,α'-(p-methoxybenzylidenedithio)diacetic acid,
α,α'-(benzylidenedithio)diacetic acid,
α,α'-(piperonylidenedithio)diacetic acid,
α,α'-(vanillylidenedithio)diacetic acid,
α,α'-(veratrylidenedithio)diacetic acid,
as well as their inorganic or organic base monosalts and disalts.

7. Use according to one of Claims 1 to 6, **characterized in that** the acid is S-carboxymethylcysteine or one of its inorganic or organic base mono- and disalts.

8. Use according to one of Claims 1 to 7, **characterized in that** the acid is used in a quantity ranging from 0.2 to 20% and preferably from 0.5 to 5% by weight with respect to the total weight of the composition.

9. Use according to one of Claims 1 to 8, **characterized in that** the composition comprises, in addition, at least one active agent chosen from the α- or β-hydroxy acids, the α- or β-keto acids and the retinoids.

10. Use according to one of Claims 1 to 9, **characterized in that** the composition comprises, in addition, at least one active agent chosen from the glycolic, lactic, malic, tartaric, citric, 2-hydroxyalkanoic, mandelic or salicylic acids, and 5-n-octanoylsalicylic acid.

11. Use according to Claim 8 or 9, **characterized in that** the active agent is present in a quantity ranging from 0.1 to 5% by weight with respect to the total weight of the composition.

12. Use according to one of Claims 1 to 11, **characterized in that** the composition contains, in addition, at least one adjuvant chosen from proteins or protein hydrolysates, amino acids, polyols, urea, sugars and sugar derivatives, vitamins, starch, vegetable extracts, essential fatty acids, ceramides and essential oils.

13. Use according to one of Claims 1 to 12, **characterized in that** the composition is an aqueous, oily or aqueous/alcoholic solution, a water-in-oil or oil-in-water emulsion, a microemulsion, an aqueous or anhydrous gel, a serum or a dispersion of vesicles, of microcapsules or of microparticles.

14. Use according to one of Claims 1 to 13, **characterized in that** the composition contains, in addition, at least one antagonist of substance P and/or of CGRP.

15. Use according to Claim 14, **characterized in that** the antagonist is a strontium salt.
